# EUROPEAN PATENT SPECIFICATION

(11) **EP 0 957 845 B1**
(45) Date of publication and mention of the grant of the patent: **17.09.2003**
(21) Application number: 96920409.8
(22) Date of filing: 21.05.1996
(51) Int. Cl.: A61F 6/06

(54) **CONTRACEPTIVE TRANSCERVICAL FALLOPIAN TUBE OCCLUSION DEVICES HAVING MECHANICAL FALLOPIAN TUBE ATTACHMENT**
TRANSCERVIKALE VERHÜTUNGSVORRICHTUNG ZUM EILEITERVERSCHLIESSEN MIT MECHANISCHER BEFESTIGUNG AM EILEITER
DISPOSITIFS CONTRACEPTIFS D'OCCLUSION DE LA TROMPE DE FALLOPE PAR LE CANAL CERVICAL, A FIXATION MECANIQUE A LA TROMPE

(30) Priority: 07.06.1995 US 475252
(43) Date of publication of application: 24.11.1999
(60) Divisional application: 03014514.8
(73) Proprietor: CONCEPTUS, INC., San Carlos, CA 94070 (US)
(72) Inventor: NIKOLCHEV, Julian, Portola Valley, CA 94028 (US); TON, Dai, San Jose, CA 95131 (US)
(74) Representative: Bayliss, Geoffrey Cyril
(86) International application number: US9607486
(87) International publication number: WO96040024

(56) References cited:
- GB-A- 2 038 186
- US-A- 3 805 767
- US-A- 3 973 560
- US-A- 4 932 421

## Description

### BACKGROUND OF THE INVENTION

### 1. Field of the Invention

The present invention relates generally to contraception, and more particularly to intrafallopian contraceptive devices, and nonsurgical methods for their delivery, are also described.

Worldwide demand exists for safe, effective methods of both contraception and permanent sterilization. Although a variety of contraception and sterilization methods are available, all of the existing methods have limitations and disadvantages. Thus, the need for additional safe, low cost, reliable methods of contraception and permanent sterilization, both in developed and less developed countries, is widely recognized.

Many presently available contraception methods require significant user involvement, and user non-compliance results in quite high rates of failure. While the theoretical effectiveness of existing contraceptives, including barrier methods and hormonal therapies, is well established, overcoming user noncompliance to improve overall efficacy has proven difficult.

One form of contraception which is less susceptible to user noncompliance is the intrauterine device (IUD). IUDs have been found to have higher rates of reliability, and are effective for a longer period of time, than most other commercially available contraceptives. Unfortunately, IUDs are also associated with serious infectious complications. For this reason, the use of IUDs within the united States has decreased dramatically. Additionally, IUDs are subject to unplanned expulsion, and must be removed due to excessive pain or bleeding in a percentage of cases, further reducing the acceptance of the IUD as a contraceptive method. Interestingly, the efficacy of copper IUDs appears to be higher than that of non-metallic IUDs. The reason for this has not been fully explained.

Commercially available options for permanent sterilization include fallopian tube ligation and vasectomy. These methods are surgical, are difficult to reverse, and are not available to many people in the world. It is common knowledge that fertilization occurs in the fallopian tubes where the sperm and ovum meet. Tubal ligation avoids this by complete occlusion of the fallopian tubes.

It has previously been proposed to reversibly occlude the fallopian tubes, for example, by *in vitro* formation of an elastomeric plug, or otherwise anchoring a device on either side of the narrowest region of fallopian tube, called the "isthmus." Such fallopian tube occlusion methods appear promising; however, an unacceptably high percentage of the non-surgical devices proposed to date have become dislodged during previous studies. Even where non-surgical intrafallopian devices have remained in place, they have been found to be only moderately effective at preventing conception.

For these reasons, it would be desirable to provide effective, reliable intrafallopian devices for contraception and sterilization. It would be particularly desirable to provide highly effective intrafallopian devices which did not require surgery for placement. It would be especially desirable if such devices and methods allowed easy placement of the device, but were less susceptible to being dislodged than previously proposed non-surgical intrafallopian devices.

### 2. Description of the Related Art

The experimental use of a stainless steel intrafallopian device is described in *Transcatheter Tubal Sterilization in Rabbits,* Penny L. Ross, RT 29 "Investigative Radiology", pp. 570-573 (1994). The experimental use of an electrolytically pure copper wire as a surgical contraceptive intrafallopian device in rats was described in "Antifertility Effect of an Intrafallopian Tubal Copper Device", D.N. Gupta, *14 Indian Journal of Experimental Biology,* pp. 316-319 (May 1976).

U.K. Patent Application Pub. No. 2,211,095 describes an uterine screw plug for blocking the fallopian tube. European Patent Application Pub. No. 0,010,812 describes a device for placement in the ovidcuts having enlargements at either end for anchoring the device. The same device appears to be described in Netherlands Patent No. 7,810,696.

The use of the tubal occlusion devices is described in "Hysteroscopic Oviduct Blocking with Formed-in-Place Silicone Rubber Plugs", Robert A. Erb, Ph.D., et al., *The Journal of Reproductive Medicine,* pp. 65-68 (August 1979). A formed-in-place elastomeric tubal occlusion device is described in U.S. Patent No. 3,805,767, issued to Erb. U.S. Patent No. 5,065,751, issued to Wolf, describesa method and apparatus for reversibly occluding a biological tube. U.S. Patent No. 4,612,924, issued to Cimber, describes an intrauterine contraceptive device which seals the mouths of the fallopian tubes.

German Patent No. 28 03 685, issued to Brundin, describes a device for plugging a body duct with a device which swells when in contact with a body fluid.

Alternative contraceptive devices are disclosed in WO 96/40024.

U.S. 3,973,560 discloses an intrauterine contraceptive device of resilient material, which may include copper. The device may be restrained in a first configuration within a tubular inserter for entry into the uterus. Upon release from the inserter, the device assumes a second configuration in a C or Omega shape and anchors resiliently against the uterine walls.

### SUMMARY OF THE INVENTION

The present invention provides intrafallopian devices, and methods for their placement to prevent conception are described. The intrafallopian devices of the present invention are transcervically delivered, resiliently anchored structures which are formed at least in part from copper to provide long term contraception, or alternatively permanent sterilization, without the need for surgical procedures or the increased bleeding, pain and risks of infection associated with intrauterine devices (IUDs).

The intrafallopian devices of the present invention generally comprise a structure having a lumen-traversing region with a helical outer surface. The helical surface is mechanically anchored by a resilient portion of the structure which is biased to form an enlarged secondary shape, preferably forming distal and proximal anchoring loops. The anchoring loops help prevent the helical outer surface from rotating out of position, and also directly deter axial motion within the fallopian tube.

The use of copper in the intrafallopian device of the present invention improves its efficacy as a contraceptive method. Devices formed from plastically deformable materials, however, are less readily restrained in the fallopian tube. Apparently, the large variation in the actual shape and dimensions of fallopian tubes does not provide reliable anchoring for a pre-formed deformable intrafallopian device. The intrafallopian device of the present invention therefore comprises a resilient structure, usually a metallic coil, which includes a copper alloy or plating, ideally comprising an alloy including at least 75% copper. The coil material typically includes beryllium, zinc, stainless steel, platinum, a shape memory alloy, such as Nitinol™, or the like. Preferably, the coil is composed of an alloy of beryllium and copper. Although the present device will generally result in occlusion, it need not completely occlude the fallopian tube to prevent the meeting of the sperm and ovum. Instead, the presence of the copper on the resilient structure is sufficient to provide effective contraception.

Conveniently, non-surgical placement of such intrafallopian devices by transcervical introduction is possible. The resilient structure is restrainable in a straight configuration, e.g., by use of a corewire, greatly facilitating and reducing the risks of introduction. Thus, the cost and dangers associated with existing surgical contraceptive and sterilization procedures are avoided

In a first aspect, a contraceptive intrafallopian device according to the present invention comprises a proximal anchor, a distal anchor, and a lumen-traversing region extending between the anchors. The lumen traversing region has a helical outer surface and a cross-section which is smaller than the cross-sections of the proximal and distal anchors.

Preferably, the lumen-traversing region comprises a resilient structure, generally having a ribbon wound over the outer surface to form the helical shape. Anchoring is enhanced by a sharp outer edge on the ribbon. As described above, at least one of the proximal anchor, the distal anchor, and the lumen-traversing region preferably comprises copper. The proximal and distal anchors generally comprise a resilient structure biased to form an enlarged secondary shape, thereby allowing the device to be restrained in a straight configuration to facilitate transcervical introduction.

In another aspect, a contraceptive intrafallopian device according to the present invention comprises a primary coil having a proximal loop, a distal loop, and an intermediate straight section between the loops. A helical ribbon is wound over at least a portion of the intermediate section, forming a helical surface to mechanically anchor the device within the fallopian tube.

The ribbon of the present intrafallopian device generally protrudes sufficiently to firmly engage the tubal wall. Preferably, the ribbon has a width in the range between .005 and .1 inch, a thickness in the range between .001 and .2 inch, and a pitch in the range between .01 and .2 inch. The overall device geometry preferably facilitates introduction and retention, but is not large or rigid enough to interfere with internal tissue movements. Usually, the device has a length in the range between 1.5 cm and 7.5 cm when in a relaxed state, while the distal loop and the proximal loop have outer diameters of at least 3 mm. Preferably, the primary coil has an outer diameter in the range between .2 mm and 5 mm.

In another aspect, a system for delivering intrafallopian contraceptive devices according to the present invention comprises a primary coil having a proximal loop, a distal loop, and an intermediate straight section between the loops. Additionally, a lumen extends from a proximal end of the proximal loop to near a distal end of the distal loop. A helical ribbon is wound over at least a portion of the intermediate section, forming a helical surface to mechanically anchor the device within the fallopian tube. A corewire is removably disposed within the lumen of the primary coil. The corewire restrains the primary coil in a straight configuration, facilitating trancervical introduction. Optionally, the corewire is threadably received by the primary coil. Alternatively, a release catheter is slidably disposed over the corewire proximally of the primary coil to restrain the primary coil while the corewire is withdrawn proximally from the fallopian tube.

The helical ribbon is anchored in the fallopian tube by the distal and proximal loops. The ribbon is set in the tubal wall while the device is restrained in a straight configuration over a corewire by torquing on the corewire. Withdrawing of the corewire then releases the anchors. The distal anchor is generally inserted into the ampulla, distal of the isthmus, while the proximal anchor is located in the ostium. These anchors prevent rotation of the device, and also help avoid axial movement.

An intrafallopian contraceptive method for use with the present invention comprises restraining a resilient contraceptive structure in a straight configuration over a corewire, where the resilient structure includes a lumen-traversing region having a helical outer surface. The resilient structure is transcervically introduced into a target region of a fallopian tube, typically in the region of the ostium, and the corewire is withdrawn from the resilient structure. The resilient structure is mechanically anchored within the fallopian tube, a portion of the resilient structure assuming an enlarged secondary shape which is larger in cross-section than the fallopian tube. Optionally, an electric current is applied through the resilient structure to the fallopian tube, thereby effecting permanent sterilization.

### BRIEF DESCRIPTION OF THE DRAWINGS

Fig. 1 illustrates a first embodiment of a contraceptive intrafallopian device according to the present invention.
Fig. 2 illustrates a primary coil used in the contraceptive intrafallopian device of Fig. 1.
Fig. 3 illustrates a secondary coil which has been imposed on a primary coil as used in the contraceptive intrafallopian device of Fig. 1.
Fig. 4 illustrates a corewire for use with the contraceptive intrafallopian device of Fig. 1.
Fig. 5 is a cross-sectional view of a contraceptive delivery system having the contraceptive intrafallopian device of Fig. 1.
Fig. 6 illustrates an alternative embodiment of the present contraceptive intrafallopian device.
Fig. 7 illustrates a primary coil used in the contraceptive intrafallopian device of Fig. 6.
Fig. 8 schematically illustrates a contraceptive delivery system including the contraceptive intrafallopian device of Fig. 6.
Figs. 9 and 10 illustrates a method of delivery of a contraceptive intrafallopian device according to the present invention.

### DETAILED DESCRIPTION OF THE SPECIFIC EMBODIMENT

The present invention encompasses a contraceptive intrafallopian device which can alternatively be used as both a permanent and a reversible means of contraception. The present contraceptive methods and devices minimize the danger of non-use which has limited the efficacy of prior art contraceptive techniques. Moreover, the location of the present devices within the fallopian tubes provides a reduced risk of the infectious complications, increased bleeding, and pelvic pain associated with intrauterine devices (IUDS). The location and the novel shape of the present intrafallopian device provides significant advantages over IUDs, which have been found to be susceptible to unplanned expulsion and removal due to excessive pain and bleeding. The present invention takes advantage of the increase in effectiveness associated with copper IUDs, providing a resilient structure including copper which may be transcervically positioned without the need for surgery.

Although the present contraceptive method is included within a group of contraceptive techniques generally referred to as fallopian tube occlusion methods, the present invention does not necessarily rely solely on blocking the fallopian tube to prevent fertilization. Instead, contraception is apparently provided by disrupting of ovum transport, the process of fertilization, and/or cleavage of the ovum. While the effect that copper has on these processes is not fully understood, it does appear that copper intrafallopian devices offer potentially significant increases in effectiveness over intrafallopian devices formed of other materials. Optionally, the present invention further encompasses devices which promote the growth of tissue within the tube to induce tubal occlusion, further inhibiting conception.

Conveniently, the present resilient structures are adapted to be releasably affixed over a corewire, the corewire restraining the resilient structure in a straight configuration. As the resilient structure has an outer diameter when in the straight configuration which is less than the inner diameter of the fallopian tube, the catheter containing the present intrafallopian device is easily transcervically introduced.

The present invention is anchored within the isthmus of the fallopian tube, overcoming the unintended expulsion of the device and the resulting failure of the contraceptive method. Such intrafallopian device expulsion has been the single greatest factor limiting the efficacy of easily positioned intrafallopian contraceptive techniques. The present intrafallopian devices are generally elongate resilient structures pre-formed into secondary shapes. These secondary shapes will preferably form anchors proximally and distally of the narrowest portion of the fallopian tube, called the isthmus. The secondary shape must have a larger outer diameter than the inner diameter of the isthmus.

The present device is generally readily removed by snaring the resilient structure near the proximal end and pulling proximally on the resilient structure, thereby straightening the resilient structure and allowing it to be withdrawn without injuring the fallopian tube. Alternatively, an electrical current is applied to the device after it is positioned within the fallopian tube, providing permanent sterilization.

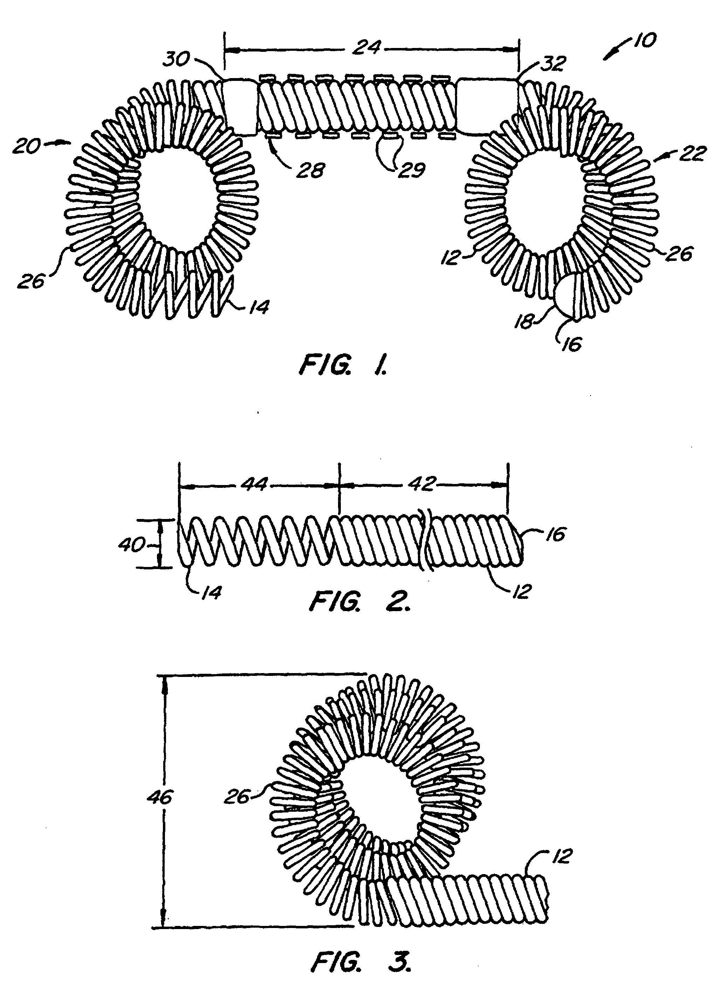

Referring now to Fig. 1, a first embodiment of the present contraceptive intrafallopian device 10 is formed from a resilient primary coil 12. Primary coil 12 has a proximal end 14 and a distal end 16, the latter having an atraumatic endcap 18. Primary coil 12 further includes three portions: a proximal anchor portion 20, a distal anchor portion 22, and a lumen-traversing region 24. Proximal and distal anchors 20,22 are biased to form anchoring loops 26, as described hereinbelow.

Lumen-traversing region 24 comprises a substantially straight portion of primary coil 12. A ribbon 28 is wound over the outer surface of primary coil 12 to provide a helical shape. Ribbon 28 includes sharp outer edges 29, which firmly anchor lumen-traversing region 24 in the fallopian tube wall when torque is applied to intrafallopian device 10. The ribbon is preferably formed of a high strength biocompatible metal, ideally being stainless steel. The ribbon is attached to primary coil 12 at a proximal joint 30 and a distal joint 32, which may be formed of solder, heat-shrink tubing, or the like.

Referring now to Fig. 2, primary coil 12 is most easily formed in a straight configuration as a cylindrical coil or spring, preferably having an outer diameter in the range from 0.13 mm (.005 inch) to 13 mm (.05 inch), and having a length in the range from 20 mm to 150 mm. Ideally, primary coil 12 has an outer diameter in the range from 0.25 mm (.01 inch) to 1.3 mm (.05 inch) and a length in the range from 30 mm to 125 mm.

Preferably, primary coil 12 is formed from a beryllium copper alloy wire. Beryllium copper provides the resilience necessary to avoid expulsion of the device, and also provides the increased effectiveness of a copper contraceptive intrafallopian device. Such a beryllium copper wire will typically have a diameter from 0.051 mm (.002 inch) to 0.25 mm (.01 inch). To provide the increased efficacy of a copper intrafallopian device, primary coil 12 preferably comprises an alloy including 75% copper. Alternatively, primary coil 12 is formed from a resilient metal, such as stainless steel, platinum, a shape memory alloy, or the like. If such materials are used, primary coil 12 is preferably plated with copper or a copper alloy or otherwise has copper attached.

Primary coil 12 includes a body winding 42 and a thread winding 44. Body winding 42 is formed with the minimum possible pitch to increase the stiffness of primary coil 12. Thread winding 44 will typically comprise from 0.1 cm to 2 cm adjacent to proximal end 14, and will have a pitch roughly twice that of body winding 42.

Referring now to Fig. 3, the proximal and distal anchors are formed by imposing a bent secondary shape on selected portions of primary coil 12. The secondary shape preferably comprises loops 26 formed by bending primary coil 12, and heat treating the primary coil while it is bent. A wide variety of secondary shapes may be used, including sinusoidal curves, alternating loops, or loops separated by straight sections so as to form a "flower coil," as more fully described in copending U.S. Patent Application Serial No. 08/474.779 , (Attorney Docket No. 16355-24 and published as WO96/40023A on 19/12/96). In all cases, the bent secondary shape should have an outer cross-section 46 which is larger than the fallopian tube to provide effective anchoring.

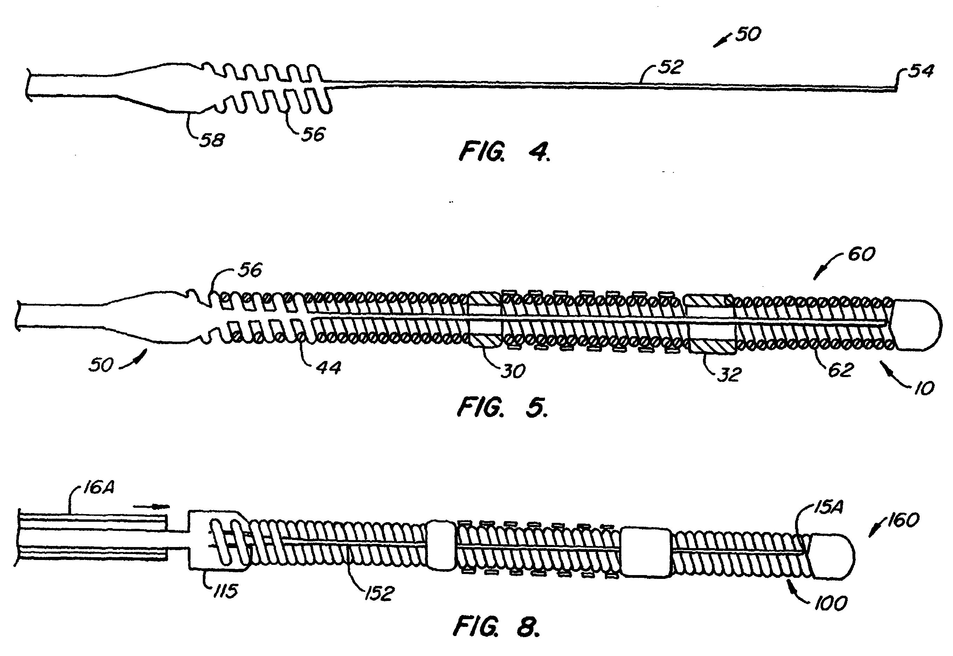

Referring now to Fig. 4, a corewire 50 for use with intrafallopian device 10 (Fig. 1) comprises a resilient wire 52 which tapers towards a distal end 54. Wire 52 is sufficiently stiff to restrain intrafallopian device 10 in a straight configuration, typically comprising stainless steel, platinum, or the like. A short section of coil forms corewire threads 56 attached at threadjoint 58. Threads 56 match the windings and pitch of threadwindings 44 of primary coil 12.

Referring now to Fig. 5, an intrafallopian contraceptive system 60 comprises corewire 50 inserted within a lumen 62 through intrafallopian device 10. Intrafallopian device 10 is releasably attached by engaging thread windings 44 with threads 56. Thus, intrafallopian device 10 is disengaged by torquing a proximal end of corewire 50 once intrafallopian device 10 is in position.

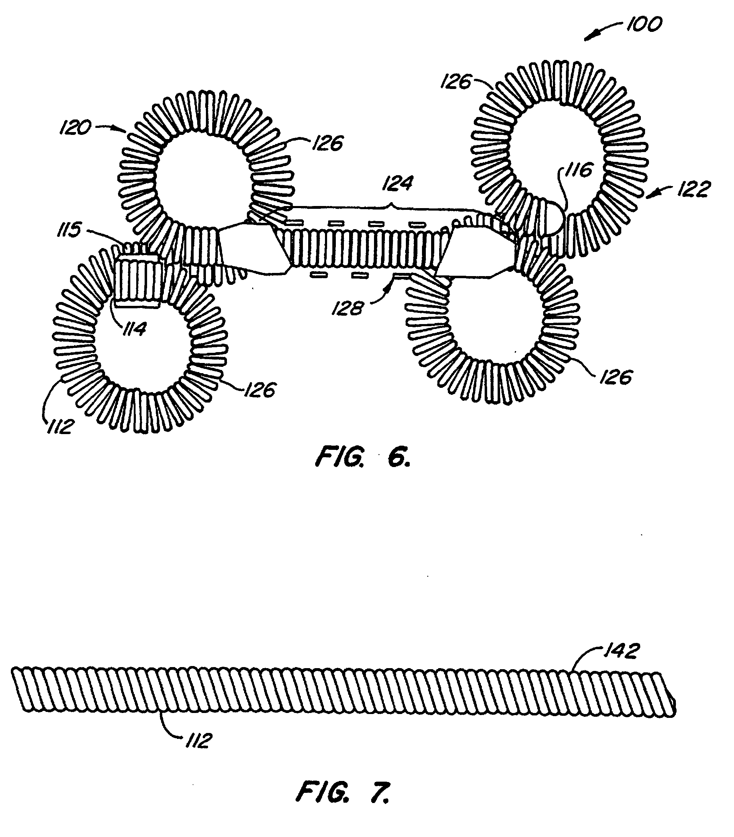

Referring now to Fig. 6, an alternative embodiment of the present intrafallopian device is again formed from a resilient primary coil 112 having a proximal end 114 and a distal end 116. The former includes a friction fitting 115. Primary coil 112 again includes three portions: a proximal anchor portion 120, a distal anchor portion 122, and a lumen-traversing region 124. Proximal and distal anchors 120, 122 are here biased to form opposed anchoring loops 26, thereby increasing the relaxed overall cross-section of the proximal and distal anchors. A ribbon 128 is wound over the outer surface of primary coil 112 to provide a helical shape, as described above.

Referring now to Fig. 7, primary coil 112 comprises a uniform body winding 142. The secondary shape is imposed on the straight cylindrical coil as opposed loops 126, or alternatively as multiple loops of a flower coil.

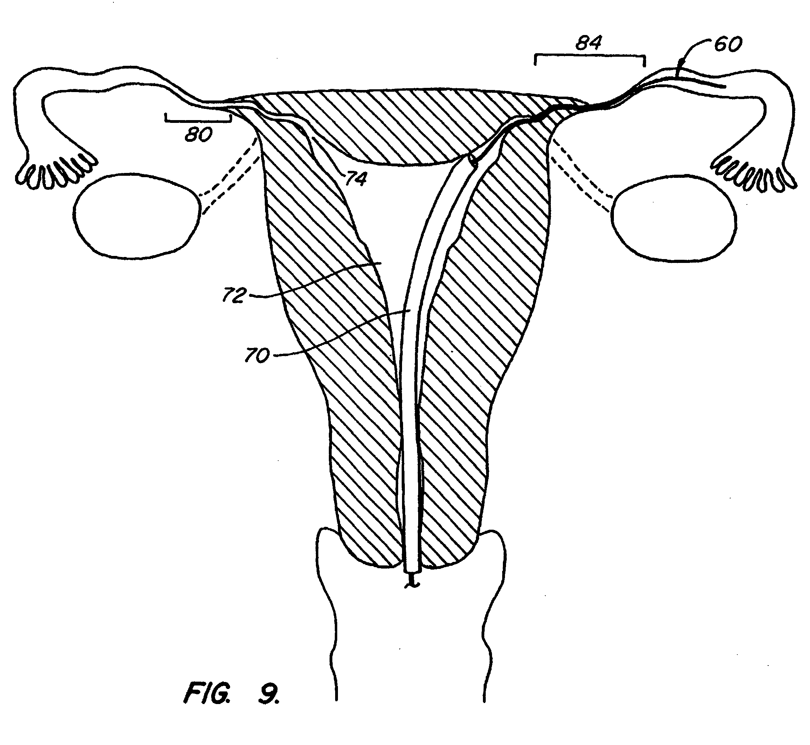

Referring now to Fig. 8, an intrafallopian contraceptive system using alternative intrafallopian device 100 includes a corewire 152 which tapers towards a distal end 154. Friction fitting 115 fittingly engages corewire 152, which restrains primary coil 112 in a straight configuration. A release catheter 164 is slidably disposed over corewire 152 proximally of alternative intrafallopian device 100, allowing the device to be released by withdrawing corewire 152 relative to the release catheter.

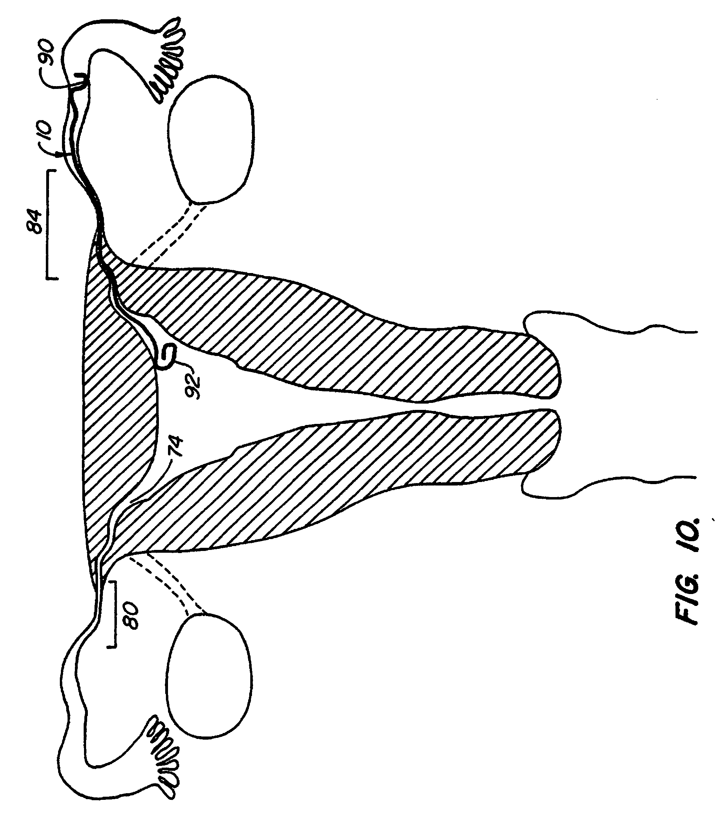

Use of the present contraceptive intrafallopian device will be described with reference to Figs. 9 and 10. A uterine introducer canula 70 is inserted transcervically through a uterus 72 to the region of an ostium 74. Alternatively, a hysteroscope may be used in place of canula 70.

Intrafallopian contraceptive system 60 is advanced distally of introducer cannula 70 and manuevered through the fallopian tube, preferably until intrafallopian device 10 extends distally of the isthmus. Optionally, intrafallopian contraceptive system 60 is self-guided, with corewire 52 bent near distal end 54 to assist intraluminal manuevering. Alternatively, a guide wire and catheter are advanced into the fallopian tube first, and the guide wire is replaced with intrafallopian contraceptive system 60. In either case, the intrafallopian device is axially positioned with lumen-traversing region 24 within a target region 84 adjacent to isthmus 80. Preferably, at least one loop of distal anchor 22 is distal of target region 84, and at least one loop of proximal anchor 20 is proximal of target region 84 to form the distal and proximal anchor bends.

Once intrafallopian device 10 is properly positioned, corewire 50 is torqued to set ribbon 28 in the tubal wall. The corewire may then be unthreaded from intrafallopian device 10 by rotating the corewire in the opposite direction, disengaging threads 56 from thread windings 44. The corewire is then free to slide proximally, releasing the primary coil. As the distal end of the primary coil is released, a distal anchor bend 90 is formed. Similarly, a proximal loop forms a proximal anchor bend 92. The anchor bends help to axially restrain the device within the fallopian tube, and also prevent rotation around the helical shape of lumen-traversing region 24. As seen in Fig. 10, the loops need not assume their relaxed form to provide effective distal or proximal anchors.

The present invention further encompasses permanent sterilization by passing a current through the corewire to the intrafallopian device prior to withdrawing the corewire. Fallopian tube tissue in contact with the intrafallopian device is dessechated, and thus attached to the present intrafallopian device. This action also causes permanent tubal damage, leading to the formation of scar tissue which encapsulates the intrafallopian device and causes permanent occlusion of the tubal lumen. Clearly, the corewire/primary coil interface must be conductive to allow the present non-surgical method of permanent sterilization.

In conclusion, the present invention provides a contraceptive intrafallopian device which may be positioned without surgery. While the above is a complete description of the preferred embodiments of the invention, various alternatives, modifications, and equivalents may be used. For example, a wide variety of secondary shapes, including open loops, continuous bends, sinusoidal curves, or the like, may be imposed on the primary coil. Therefore, the above description should not be taken as limiting the scope of the invention, which is defined instead solely by the appended claims.

## Claims

1. A contraceptive device (10,100) comprising:
a proximal anchor (20,120) having a proximal cross-section;
a distal anchor (22,122) having a distal cross-section; and
a lumen-traversing region (24,124) extending between the proximal anchor (20,120) and the distal anchor (22,122), the lumen traversing region comprising a resilient structure;
**characterised in that** the device is an intrafallopian device, a ribbon (28,128) is wound over the outer surface of the resilient structure, and the lumen traversing region (24,124) has a helical outer surface and a helical cross-section which is smaller than both the proximal cross-section and the distal cross-section.

2. An intrafallopian contraceptive device (10,100) as claimed in claim 1, wherein the ribbon (28,128) includes a sharp outer edge (29).

3. An intrafallopian contraceptive device (10,100) as claimed in claim 1, wherein at least one of the proximal anchor (20,120), the distal anchor (22,122), and the lumen-traversing region (24,124) comprises copper.

4. An intrafallopian contraceptive device (10,100) as claimed in claim 1, wherein at least one of the proximal anchor (20,120) and the distal anchor (22,122) comprises a resilient structure biased to form a secondary shape.

5. An intrafallopian contraceptive device (10,100) as claimed in claim 4, wherein the resilient structure comprises a primary coil (12,120).

6. An intrafallopian contraceptive device (10,100) as claimed in claim 5, wherein the primary coil (12,120) comprises a material selected from the group consisting of beryllium, stainless steel, platinum, and shape memory alloy.

7. An intrafallopian contraceptive device (10,100) as claimed in claim 6, wherein the primary coil (12,120) comprises an alloy including beryllium and copper.

8. An intrafallopian contraceptive device (10,100) as claimed in claim 5, wherein the primary coil (12,120) comprises an alloy including at least 75% copper.

9. An intrafallopian contraceptive device (10,100) as claimed in claim 1, wherein a lumen extends from a proximal end of the proximal anchor (20,120) to near a distal end of the distal anchor (22), and wherein the intrafallopian contraceptive device is restrainable in a straight configuration by a corewire (50) within the lumen.

10. An intrafallopian contraceptive device (10,100) as claimed in claim 1, comprising:
a primary coil (12,120) having a distal loop, a proximal loop, and an intermediate straight section between the distal loop and the proximal loop; and
a helical ribbon wound (28,128) over at least a portion of the intermediate section.

11. An intrafallopian contraceptive device (10,100) as claimed in claim 10, wherein the ribbon (28,128) has a width in the range between 0.13mm and 2.5mm (.005 and .1 inch).

12. An intrafallopian contraceptive device (10,100) as claimed in claim 11, wherein the ribbon (28,128) has a thickness in the range between 0.03mm and 5.1mm (.001 and .2 inch).

13. An intrafallopian contraceptive device (10,100) as claimed in claim 10, wherein the ribbon (28,128) has a pitch in the range between 0.25mm and 5.1mm (.01 and .2 inch).

14. An intrafallopian contraceptive device (10,100) as claimed in claim 10, wherein the device has a length in the range between 1.5cm and 7.5cm when in a relaxed state.

15. An intrafallopian contraceptive device (10,100) as claimed in claim 10, wherein the device comprises copper.

16. An intrafallopian contraceptive device (10,100) as claimed in claim 15, wherein the primary coil (12,120) comprises a material selected from the group consisting of beryllium, stainless steel, platinum, and shape memory alloy.

17. An intrafallopian contraceptive device (10,100) as claimed in claim 16, wherein the primary coil (12,120) comprises an alloy including beryllium and copper.

18. An intrafallopian contraceptive device (10,100) as claimed in claim 10, wherein the primary coil (12,120) includes a lumen which extends from a proximal end of the proximal loop to near the distal end of the distal loop.

19. An intrafallopian contraceptive device (10,100) as claimed in claim 10, wherein the primary coil (12,120) has an outer diameter in the range between .2mm and 5mm.

20. An intrafallopian contraceptive device (10,100) as claimed in claim 10, wherein the distal loop and the proximal loop have outer diameters of at least 3mm when in a relaxed state.

21. A contraceptive system (60) comprising:
a primary coil (12,120) having a distal loop, a proximal loop, and an intermediate straight section between the distal loop and the proximal loop;
**characterised in that** the device is an intrafallopian device, the primary coil further comprises a lumen from a proximal end of the proximal loop to near a distal end of the distal loop;
a helical ribbon (28,128) is wound over at least a portion of the intermediate section so as to provide a helical outer surface and a helical cross-section which is smaller than that of the proximal and distal loops; and
a corewire (50,152) is removably disposed within the lumen of the primary coil (12,120), the corewire (50,152) restraining the primary coil (12,120) in a straight configuration.

22. An intrafallopian contraceptive system (60) as claimed in claim 21, wherein the primary coil (12,120) comprises copper.

23. An intrafallopian contraceptive system (60) as claimed in claim 21, wherein the corewire (50,152) is threadably received by the primary coil (12,120).

24. An intrafallopian contraceptive system (60) as claimed in claim 21, further comprising a release catheter slidably disposed over the corewire (50,152) proximally of the primary coil (12,120), the release catheter having a distal primary coil engaging surface for restraining the primary coil (12,120) while the corewire (50,152) is withdrawn proximally.

## Patentansprüche

1. Kontrazeptive Vorrichtung (10,100) umfassend:
einen proximalen Anker (20, 120) mit einem proximalen Querschnitt,
einen distalen Anker (22, 122) mit einem distalen Querschnitt; und
einen Lumen-querenden Bereich (24, 124), der sich zwischen dem proximalen Anker (20, 120) und dem distalen Anker (22, 122) erstreckt, wobei der Lumen-querende Bereich eine elastische Struktur umfasst;
**dadurch gekennzeichnet, dass** die Vorrichtung eine in den Eileiter einzusetzende Vorrichtung ist, ein Band (28, 128) um die Außenoberfläche der elastischen Struktur herumgewickelt ist und der Lumen-querende Bereich (24, 124) eine gewendelte Außenoberfläche und einen gewendelten Querschnitt hat, der kleiner ist als sowohl der proximale Querschnitt als auch der distale Querschnitt.

2. In den Eileiter einzusetzende kontrazeptive Vorrichtung (10, 100) nach Anspruch 1, worin das Band (28, 128) einen scharfen Außenrand (29) aufweist.

3. In den Eileiter einzusetzende kontrazeptive Vorrichtung (10, 100) nach Anspruch 1, worin zumindest einer des proximalen Ankers (20, 120), des distalen Ankers (22, 122) und des Lumen-querenden Bereichs (24, 124) Kupfer aufweist.

4. In den Eileiter einzusetzende kontrazeptive Vorrichtung (10, 100) nach Anspruch 1, worin zumindest einer des proximalen Ankers (20, 120) und des distalen Ankers (22, 122) eine elastische Struktur umfasst, die zur Bildung einer sekundären Form vorgespannt ist.

5. In den Eileiter einzusetzende kontrazeptive Vorrichtung (10, 100) nach Anspruch 4, worin die elastische Struktur eine Primärwicklung (12, 120) umfasst.

6. In den Eileiter einzusetzende kontrazeptive Vorrichtung (10, 100) nach Anspruch 5, worin die Primärmrircklung (12, 120) ein Material umfasst, das aus der Gruppe ausgewählt ist, die aus Beryllium, rostfreiem Stahl, Platin und Formgedächtnislegierung besteht.

7. In den Eileiter einzusetzende kontrazeptive Vorrichtung (10, 100) nach Anspruch 6, worin die Primärwicklung (12 ,120) eine Beryllium und Kupfer enthaltende Legierung aufweist.

8. In den Eileiter einzusetzende kontrazeptive Vorrichtung (10, 100) nach Anspruch 5, worin die Primärwicklung (12, 120) eine zumindest 75 % Kupfer enthaltende Legierung aufweist.

9. In den Eileiter einzusetzende kontrazeptive Vorrichtung (10, 100) nach Anspruch 1, worin sich ein Lumen von einem proximalen Ende des proximalen Ankers (20, 120) zu nahe einem distalen Ende des distalen Ankers (22) erstreckt, und worin die intrafallopiane kontrazeptive Vorrichtung durch einen Kerndraht (50) in dem Lumen in einer geraden Konfiguration zurückhaltbar ist.

10. In den Eileiter einzusetzende kontrazeptive Vorrichtung (10, 100) nach Anspruch 1, umfassend:
eine Primärwicklung (12, 120) mit einer distalen Schleife, einer proximalen Schleife und einem zwischenliegenden geraden Abschnitt zwischen der distalen Schleife und der proximalen Schleife ; und
eine gewendelte Bandwindung (28, 128) über zumindest einem Teil des zwischenliegenden Abschnitts.

11. In den Eileiter einzusetzende kontrazeptive Vorrichtung (10, 100) nach Anspruch 10, worin das Band (28, 128) eine Breite im Bereich zwischen 0,13 mm und 2,5 mm (0,005 und 0,1 Zoll) hat.

12. In den Eileiter einzusetzende kontrazeptive Vorrichtung (10, 100) nach Anspruch 11, worin das Band (28, 128) eine Dicke im Bereich zwischen 0,03 und 5,1 mm (0,001 und 0,2 Zoll hat).

13. In den Eileiter einzusetzende kontrazeptive Vorrichtung (10, 100) nach Anspruch 10, worin das Band (28, 128) eine Steigung im Bereich zwischen 0,25 mm und 5,1 mm (0,01 und 0,2 Zoll) hat.

14. In den Eileiter einzusetzende kontrazeptive Vorrichtung (10, 100) nach Anspruch 10, worin die Vorrichtung, im entspannten Zustand, eine Länge im Bereich zwischen 1,5 cm und 7,5 cm hat.

15. In den Eileiter einzusetzende kontrazeptive Vorrichtung (10, 100) nach Anspruch 10, worin die Vorrichtung Kupfer aufweist.

16. In den Eileiter einzusetzende kontrazeptive Vorrichtung (10, 100) nach Anspruch 15, worin die Primärwircklung (12, 120) ein Material auweist, das aus der Gruppe gewählt ist, die aus Beryllium, rostfreiem Stahl, Platin und Formgedächtnislegierung besteht.

17. Intrafallopiane kontrazeptive Vorrichtung (10, 100) nach Anspruch 16, worin die Primärwicklung (12, 120) eine Beryllium und Kupfer enthaltende Legierung aufweist.

18. In den Eileiter einzusetzende kontrazeptive Vorrichtung (10, 100) nach Anspruch 10, worin die Primärwicklung (12, 120) ein Lumen enthält, das sich von einem proximalen Ende der proximalen Schleife bis nahe dem distalen Ende der distalen Schleife erstreckt.

19. ln den Eileiter einzusetzende kontrazeptive Vorrichtung (10, 100) nach Anspruch 10, worin die Primärwicklung (12, 120) einen Außendurchmesser im Bereich zwischen 0,2 und 5 mm aufweist.

20. In den Eileiter einzusetzende kontrazeptive Vorrichtung (10, 100) nach Anspruch 10, worin die distale Schleife und die proximale Schleife, im entspannten Zustand, Außendurchmesser von zumindest 3 mm aufweisen.

21. Kontrazeptives System (60) umfassend:
eine Primärwicklung (12, 120) mit einer distalen Schleife, einer proximalen Schleife und einem zwischenliegenden geraden Abschnitt zwischen der distalen Schleife und der proximalen Schleife;
**dadurch gekennzeichnet, dass** die Vorrichtung eine in den Eileiter einzusetzende Vorrichtung ist, wobei die Primärwicklung ferner ein Lumen von einem proximalen Ende der proximalen Schleife bis nahe einem distalen Ende der distalen Schleife aufweist;
ein gewendeltes Band (28, 128) über zumindest einen Teil des zwischenliegenden Abschnitts gewickelt ist, um eine gewendelte Außenoberfläche und einen gewendelten Querschnitt vorzusehen, der kleiner ist als jener der proximalen und distalen Schleifen; und
ein Kerndraht (50, 152) in dem Lumen der Primärwicklung (12, 120) enfernbar angeordnet ist, wobei der Kerndraht (50, 152) die Primärwicklung (12, 120) in einer geraden Konfiguration zurückhält.

22. In den Eileiter einzusetzendes kontrazeptives System (60) nach Anspruch 21, worin die Primärwicklung (12,120) Kupfer aufweist.

23. In den Eileiter einzusetzendes kontrazeptives System (60) nach Anspruch 21, worin der Kerndraht (50, 152) von der Primärwicklung (12, 120) schraubbar aufgenommen ist.

24. In den Eileiter einzusetzendes kontrazeptives System (60) nach Anspruch 21, das ferner einen Ausgabekatheter aufweist, der gleitend über dem Kerndraht (50, 152) proximal der Primärwicklung (12, 120) angeordnet ist, wobei der Ausgabekatheter eine distale Primärwicklungs-Eingriffsoberfläche aufweist, um die Primäwicklung (12, 120) zurückzuhalten, während der Kerndraht (50, 152) proximal abgezogen wird,

## Revendications

1. Dispositif contraceptif (10, 100) comportant :
une pièce d'ancrage proximal (20, 120) ayant une section transversale poximale ;
une pièce d'ancrage distal (22, 122) ayant une section transversale distale ; et
une région (24, 124) de passage d'une lumière s'étendant entre la pièce d'ancrage proximal (20, 120) et la pièce d'ancrage distal (22, 122), la région parcourue par une lumière comportant une structure élastique ;
**caractérisé en ce que** le dispositif est un dispositif intrafallopien, un ruban (23, 128) est enroulé sur la surface extérieure de la structure élastique, et la région (24, 124) parcourue par une lumière présente une surface extérieure hélicoïdale et une section transversale hélicoïdale qui est plus petite à la fois que la section transversale proximale et que la section transversale distale.

2. Dispositif contraceptif intrafallopien (10, 100) selon la revendication 1, dans lequel le ruban (28, 128) comporte un bord extérieur effilé (29).

3. Dispositif contraceptif intrafallopien (10, 100) selon la revendication 1, dans lequel au moins l'une de la pièce d'ancrage proximal (20, 120), de la pièce d'ancrage distal (22, 122) et de la région (24, 124) parcourue par une lumière comprend du cuivre.

4. Dispositif contraceptif intrafallopien (10, 100) selon la revendication 1, dans lequel au moins l'une de la pièce d'ancrage proximale (20, 120) et de la pièce d'ancrage distale (22, 122) comprend une structure élastique sollicitée pour façonner une forme secondaire.

5. Dispositif contraceptif intrafallopien (10, 100) selon le revendication 4, dans lequel la structure élastique comporte un enroulement principal (12, 120).

6. Dispositif contraceptif intrafallopien (10, 100) selon la revendication 5, dans lequel l'enroulement principal (12, 120) comprend une matière choisie dans le groupe constitué du béryllium, de l'acier inoxydable, du platine et d'un alliage à mémoire de forme.

7. Dispositif contraceptif intrafallopien (10, 100) selon la revendication 6, dans lequel l'enroulement principal (12, 120) comprend un alliage contenant du béryllium et du cuivre.

8. Dispositif contraceptif intrafallopien (10, 100) selon la revendication 5, dans lequel l'enroulement principal (12, 120) comprend un alliage contenant au moins 75% de cuivre.

9. Dispositif contraceptif intrafallopien (10, 100) selon la revendication 1, dans lequel une lumière s'étend d'une extrémité proximale de la pièce d'ancrage proximal (20, 120) jusqu'à proximité d'une extrémité distale de la pièce d'ancrage distal (22), et dans lequel le dispositif contraceptif intrafallopien peut être retenu dans une configuration droite par un fil métallique (50) d'âme à l'intérieur de la lumière.

10. Dispositif contraceptif intrafallopien (10, 100) selon la revendication 1, comportant :
un enroulement principal (12, 120) ayant une boucle distale, une boucle proximale et un tronçon droit intermédiaire entre la boucle distale et la boucle proximale ; et
un ruban hélicoïdal (28, 128) enroulé sur au moins une partie du tronçon intermédiaire.

11. Dispositif contraceptif intrafallopien (10, 100) selon la revendication 10, dans lequel le ruban (28, 128) a une largeur dans la plage comprise entre 0,13 et 2,5 mm (0,005 et 0,1 inch).

12. Dispositif contraceptif intrafallopien (10, 100) selon la revendication 11, dans lequel le ruban (26, 128) a une épaisseur dans la plage comprise entre 0,03 mm et 5,1 mm (0,001 et 0,2 inch).

13. Dispositif contraceptif intrafallopien (10, 100) selon la revendication 10, dans lequel le ruban (28, 128) a un pas dans la plage comprise entre 0,25 mm et 5,1 mm (0,01 et 0,2 inch).

14. Dispositif contraceptif intrafallopien (10, 100) selon la revendication 10, dans lequel le dispositif a une longueur dans la plage comprise entre 1,5 cm et 7,5 cm lorsqu'il est dans un état relâché.

15. Dispositif contraceptif intrafallopien (10, 100) selon la revendication 10, dans lequel le dispositif comprend du cuivre.

16. Dispositif contraceptif intrafallopien (10, 100) selon la revendication 15, dans lequel l'enroulement principal (12, 120) comprend une matière choisie dans le groupe constitué du béryllium, de l'acier inoxydable, du platine et d'un alliage à mémoire de forme.

17. Dispositif contraceptif intrafallopien (10, 100) selon la revendication 16, dans lequel l'enroulement principal (12, 120) comprend un alliage contenant du béryllim et du cuivre.

18. Dispositif contraceptif intrafallopien (10, 100) selon la revendication 10, dans lequel l'enroulement principal (12, 120) comprend une lumière qui s'étend d'une extrémité proximale de la boucle proximale jusqu'à proximité de l'extrémité distale de la boucle distale.

19. Dispositif contraceptif intrafallopien (10, 100) selon la revendication 10, dans lequel l'enroulement principal (12, 120) a un diamètre extérieur dans la plage comprise entre 0,2 mm et 5 mm.

20. Dispositif contraceptif intrafallopien (10, 100) selon la revendication 10, dans lequel la boucle distale et la boucle proximale ont des diamètres extérieurs d'au moins 3 mm lorsqu'elles sont dans un état relâché.

21. Système contraceptif (60) comportant :
un enroulement principal (12, 120) ayant une boucle distale, une boucle proximale et un tronçon droit intermédiaire entre la boucle distale et la boucle proximale ;
**caractérisé en ce que** le dispositif est un dispositif intrafallopien, la boucle principale comporte en outre une lumière allant d'une extrémité proximale de la boucle proximale jusqu'à proximité d'une extrémité distale de la boucle distale ;
un ruban hélicoïdal (28, 128) est enroulé sur au moins une partie du tronçon intermédiaire afin de procurer une surface extérieure hélicoïdale et une section transversale hélicoïdale qui est plus petite que celle des boucles proximale et distale ; et
un fil métallique d'âme (50, 152) est disposé de façon amovible dans la lumière de l'enroulement principal (12, 120), le fil métallique d'âme (50, 152) retenant l'enroulement principal (12, 120) dans une configuration droite.

22. Système contraceptif intrafallopien (60) selon la revendication 21, dans lequel l'enroulement principal (12, 120) comprend du cuivre.

23. Système contraceptif intrafallopien (60) selon la revendication 21, dans lequel le fil métallique d'âme (50, 152) est reçu par vissage par l'enroulement principal (12, 120).

24. Système contraceptif intrafallopien (60) selon la revendication 21, comportant en outre un cathéter de libération disposé de façon coulissante sur le fil métallique d'âme (50, 152) du côté proximal de l'enroulement principal (12, 120) le cathéter de libération ayant une surface distale d'entrée en prise avec l'enroulement principal pour retenir l'enroulement principal (12, 120) tandis que le fil métallique d'âme (50, 152) est retiré du côté proximal.
